Europäisches Patentamt

(19) European Patent Office

Office européen des brevets

(11) EP 0 797 462 B1

## (12) FASCICULE DE BREVET EUROPEEN

(45) Date de publication et mention
de la délivrance du brevet:
**08.07.1998 Bulletin 1998/28**

(21) Numéro de dépôt: **95941063.0**

(22) Date de dépôt: **30.11.1995**

(51) Int. Cl.$^6$: **A61M 5/172**

(86) Numéro de dépôt international:
**PCT/EP95/04722**

(87) Numéro de publication internationale:
**WO 96/17637 (13.06.1996 Gazette 1996/27)**

(54) **DISPOSITIF POUR LA SURVEILLANCE DU DEBIT D'UNE PERFUSION INTRAVEINEUSE**

VORRICHTUNG ZUR ÜBERWACHUNG DES INFUSIONSFLUSSES

INTRAVENOUS INFUSION FLOW RATE MONITORING DEVICE

(84) Etats contractants désignés:
**AT BE CH DE DK ES FR GB GR IE IT LI MC NL PT
SE**

(30) Priorité: **07.12.1994 LU 88565**

(43) Date de publication de la demande:
**01.10.1997 Bulletin 1997/40**

(73) Titulaire:
**MIDEX MARKETING LIMITED
Dublin II (IE)**

(72) Inventeur: **MOLKO, Albert
L-5250 Sandweiler (LU)**

(74) Mandataire: **Schmitt, Armand et al
Office de brevets,
Ernest T. Freylinger,
321, route d'Arlon,
B.P. 48
8001 Strassen (LU)**

(56) Documents cités:
**EP-A- 0 002 775        EP-A- 0 189 491
EP-A- 0 441 323        EP-A- 0 595 474
DE-A- 3 806 800**

**Description**

La présente invention concerne un dispositif pour la surveillance du débit d'une perfusion intraveineuse.

De nombreuses substances médicamenteuses sont administrées par voie de perfusion intraveineuse. La toxicité, les impératifs de régularité dans la répartition chronologique de la dose, l'état physique du malade, font qu'il est souhaitable, voire indispensable de surveiller le débit d'une perfusion intraveineuse.

La perfusion par gravité, avec ajustage manuel du débit par écrasement progressif de la tubulure de perfusion et surveillance visuelle du débit à l'aide d'une ampoule compte-gouttes, est encore aujourd'hui la méthode de perfusion la plus utilisée. Il va sans dire que l'opération d'ajustage du débit est loin d'être précise. L'infirmière doit d'abord calculer, en fonction de la durée de perfusion souhaitée et du nombre de gouttes par unité de volume (qui dépend du liquide de perfusion et de l'ampoule compte-gouttes utilisée), l'intervalle de temps séparant deux gouttes. Ensuite elle doit ajuster le débit par écrasement de la tubulure de perfusion en chronométrant à l'aide d'une montre les gouttes dans l'ampoule compte-gouttes. Pendant toute la durée de l'infusion elle doit régulièrement repasser pour vérifier visuellement le volume restant, ainsi que le débit de gouttes dans l'ampoule compte-gouttes, afin de conclure subjectivement si la progression de l'infusion est plus ou moins acceptable. Si elle soupçonne un retard ou une avance, elle ajuste le débit en ayant largement recours à son flair, plutôt que de refaire des calculs ou un chronométrage des gouttes.

Si on veut avoir une précision plus élevée du réglage de débit et avoir l'assurance que le débit de perfusion est respecté pendant toute la durée de la perfusion, on doit avoir recours à des dispositifs de perfusion à pompes de dosage (appelées aussi pompes péristaltiques) qui sont conçues pour délivrer un débit quasi constant. Mais ces dispositifs présentent malheureusement des inconvénients qui en imitent l'usage. D'abord, la consommation électrique des pompes de dosage est loin d'être négligeable. Alimentés par une batterie, l'autonomie de ces dispositifs est par conséquent restreinte. Leur poids et leur encombrement appréciables font que ces dispositifs ne peuvent guère être utilisés pour des patients non alités. Les dispositifs de perfusion intraveineuse à pompes de dosage imposent aussi l'usage de tubulures spéciales (tubes souples en silicone). Or, le coût de ces tubulures spéciales est beaucoup plus élevé que celui de tubulures standard, et leur approvisionnement complique la gestion du matériel consommable. L'emploi d'une pompe de dosage impose en outre le contrôle de la pression de refoulement. Cette pression dépend de la contre-pression au point d'injection et est par conséquent un paramètre très sensible aux moindres anomalies. Il sera encore noté que la mesure même de la pression de refoulement des pompes est problématique. En effet, vu que la tubulure doit rester stérile, les capteurs de pression ne peuvent être qu'externes à la tubulure. Or, il n'est pas évident de mesurer une surpression à travers une tubulure en plastique.

Les dispositifs de perfusion intraveineuse à pompes de dosage qui sont aujourd'hui sur le marché sont des automates complexes munis de systèmes de contrôle et de réglage sophistiqués, qui sont souvent à l'origine d'alarmes intempestives. Dans ces systèmes sophistiqués de contrôle et de réglage, on peut intégrer un dispositif de contrôle du débit de perfusion. Un tel dispositif de contrôle du débit comprend un capteur électro-optique, du type à barrière optique, qui est associé à une ampoule compte-gouttes pour détecter la présence, respectivement l'absence d'une goutte. Le signal du capteur est traité dans une unité de comptage et de chronométrage. Une unité de calcul surveille le débit de gouttes, pour déclencher une alarme acoustique et/ou visuelle et/ou pour intervenir dans le système de réglage de la pompe de dosage lorsque ce débit de gouttes présente un écart positif ou négatif par rapport à une valeur de consigne.

Un tel dispositif de surveillance de débit est par exemple décrit dans le document EP-A-0441323. Pour augmenter la sensibilité de la détection on propose dans ce document de remplacer la photodiode par une sorte de caméra miniature qui permet de détecter une image de la goutte. Il va sans dire qu'une telle solution ne diminue ni le prix du dispositif, ni son encombrement, ni sa consommation électrique.

Il serait aussi intéressant d'utiliser des dispositifs de contrôle du débit de perfusion pour surveiller des perfusions par gravité. Or, si pour les automates de perfusion complexes et chers, le prix du dispositif de contrôle de débit joue un rôle mineur, tel n'est pas le cas si l'on veut utiliser systématiquement un dispositif de surveillance du débit autonome pour la surveillance de perfusion par gravité. Un tel appareil de surveillance de débit devrait être bon marché (vu le grand nombre d'appareils requis dans un hôpital), être très compact et consommer peu d'énergie (pour ne pas restreindre la mobilité d'un patient qui n'est pas alité), présenter un bon confort d'utilisation (afin de garantir son acceptation par le personnel médical) et garantir une sécurité d'utilisation élevée.

Ce problème complexe, qui nécessite à première vue la solution de problèmes totalement contradictoires, trouve une solution simple dans un dispositif selon l'invention.

Selon la présente invention un dispositif de surveillance de perfusions, comprend deux types d'appareils physiquement indépendants mais fonctionnellement liés, à savoir une console de programmation autonome et indépendante et une unité de surveillance autonome et indépendante.

La console de programmation comprend:

une unité d'entrée de données apte à entrer des paramètres caractéristiques de l'infusion;

sa propre unité de calcul qui est apte à préconditionner les paramètres caractéristiques de l'infusion entrés sur l'unité d'entrée de données des paramètres, et

une interface de sortie qui est apte à transformer ces paramètres préconditionnés en signaux transmissibles à distance.

Une telle console de programmation peut être utilisée pour programmer un grand nombre d'unités de surveillance autonomes, dont chacune comprend alors:

un capteur apte a être associé à une ampoule compte-gouttes pour détecter la présence, respectivement l'absence d'une goutte;

et un boîtier comprenant les unités fonctionnelles suivantes:

une unité de chronométrage et de comptage des gouttes détectées par ledit capteur;

une unité mémoire pour stocker des paramètres caractéristiques de l'infusion;

une unité d'affichage;

une unité d'alarme acoustique et/ou visuelle;

une unité de calcul apte à utiliser un algorithme de calcul, faisant intervenir comme paramètres un signal de l'unité de chronométrage et de comptage et les paramètres caractéristiques de l'infusion stockés dans l'unité de mémoire, pour déclencher une alarme acoustique et/ou visuelle;

une interface d'entrée qui est apte à reconstituer lesdits paramètres préconditionnés de façon qu'ils puissent être chargés dans ladite unité mémoire pour utilisation par ladite première unité de calcul; et

une source autonome d'alimentation électrique pour le capteur et toutes ces unités fonctionnelles intégrées dans le boîtier commun.

Selon la présente invention on associe à une perfusion déterminée une unité de surveillance autonome qui a été préprogrammée à l'aide de la console de programmation. Il en résulte qu'un hôpital peut se contenter d'acquérir une seule unité de programmation pour un grand nombre d'unités de surveillance, ce qui réduit naturellement de façon appréciable les coûts d'investissement à réaliser pour assurer par exemple une surveillance systématique de toutes les perfusions réalisées.

La dissociation physique de la fonction surveillance et de la fonction programmation a aussi un avantage sur le plan organisation et sécurité. La programmation des unités de surveillance autonomes peut se faire exclusivement par la personne qui a accès à la console de programmation. Il en résulte que l'accès à la programmation des unités de surveillance peut être réservé à du personnel qui a la compétence nécessaire. Une personne qui n'a pas accès à la console de programmation ne peut pas changer les paramètres d'une perfusion. La responsabilité en ce qui concerne la programmation des paramètres de surveillance est ainsi clairement délimitée.

L'intégration de l'unité d'entrée de données dans une console de programmation indépendante permet de concevoir cette unité d'entrée de données d'une façon très conviviale. En effet, la place disponible, le poids et la consommation électrique constituent des contraintes d'ordre secondaire pour la conception de la console de programmation, mais des contraintes d'ordre primaire pour la conception d'une unité de surveillance compacte et autonome. De plus, le prix d'une unité de programmation peut être ventilé sur un grand nombre d'unités de surveillance autonomes, ce qui permet de choisir une solution optimale du point de vue saisie des données de la perfusion, sans être obligé de faire trop attention au coût de cette solution.

La console de programmation dispose de sa propre unité de calcul qui permet de préconditionner les données entrées en vue de leur utilisation finale dans l'unité de surveillance. Cette technique de préconditionnement des paramètres entrés au niveau de la console de programmationpermet de diminuer le nombre d'opérations à exécuter par l'unité de calcul de l'unité de surveillance et dès lors d'épargner de l'énergie électrique au niveau de cette unité de surveillance autonome.

En ce qui concerne l'unité de surveillance autonome, celle-ci peut être conçue de façon très compacte car une unité conviviale d'entrée de données volumineuse n'a pas besoin d'être intégrée dans le boîtier de l'unité de surveillance autonome. Compacte et électriquement autonome, elle ne constitue pas d'obstacle à la mobilité du patient.

L'unité d'entrée de données comprend avantageusement des moyens pour entrer directement le volume de l'infusion et des moyens pour entrer directement la durée de l'infusion. Ainsi il n'y a pas de possibilité de commettre une erreur lors de la conversion de ces deux caractéristiques fondamentales dans une grandeur dérivée, par exemple le nombre de gouttes par minute. Cette conversion est alors réalisée automatiquement par une des deux unités de calcul, de préférence déjà par l'unité de calcul de la console de programmation.

L'unité d'entrée de données comprend en outre avantageusement des moyens pour entrer une marge de tolérance. Cette marge de tolérance intervient alors dans l'algorithme de calcul de l'unité de surveillance pour déterminer une bande passante autour du débit théoriquement requis, dans laquelle il n'y a pas de déclenchement d'alarme. Cette marge de tolérance permet d'éviter des alarmes lorsque les écarts détectés n'ont pas de conséquences néfastes sur le déroulement global de l'infusion. Les moyens pour entrer une marge de tolérance du débit d'infusion comprennent de préférence des moyens pour entrer une marge de tolérance distincte pour le cas d'écarts négatifs (retard) et positifs (avance). Suivant le type de perfusion ou l'état du patient, il peut en effet être recommandé de fixer une marge de tolérance plus étroite en cas d'un écart négatif qu'en cas d'un écart positif, ou vice versa. Il sera apprécié que des marges de tolérance programmables permettent une approche souple de la surveillance d'une perfusion qui évite des alarmes inutiles. On ne cherche pas à maintenir à tout prix un débit volumique théorique, rarement nécessaire dans la pratique, mais on autorise des écarts momentanés ou globaux à l'intérieur d'une fourchette d'erreur définie en fonction du type de perfusion et de l'état du patient.

Les paramètres servant à calibrer l'unité de surveillance autonome sont eux aussi entrés avantageusement par l'intermédiaire de l'unité d'entrée de données. Ces paramètres servant à la calibration caractérisent par exemple la nature du liquide de perfusion et le type de l'ampoule compte-gouttes utilisée. Ils permettent à une des deux unités de calcul, de préférence à celle de la console de programmation, de calculer le nombre de gouttes par unité de volume. Il est cependant aussi possible d'entrer directement le nombre de gouttes par unité de volume comme paramètre de calibration.

Une unité conviviale d'entrée de données, qui est bien acceptée par le personnel médical, comprend de préférence un clavier à touches dont chaque touche est dédiée à une valeur usuelle d'un paramètre caractéristique de l'infusion. L'utilisation d'un tel type de clavier est très simple et le risque d'erreurs est réduit au minimum.

Afin que l'utilisateur ait un moyen de contrôle sur l'affectation de l'unité de surveillance autonome programmée à une perfusion déterminée, l'unité d'affichage alphanumérique permet d'afficher des paramètres caractéristiques de l'infusion déduits directement des paramètres chargés dans l'unité mémoire de l'unité de surveillance autonome. Ces paramètres caractéristiques, affichés de préférence sous forme d'une barre d'état, comprennent de préférence le volume de l'infusion et la durée de l'infusion.

Dans une exécution préférentielle, l'unité d'affichage alphanumérique affiche, de préférence graphiquement sous forme d'une échelle linéaire, l'écart relatif positif ou négatif entre le nombre de gouttes détectées pendant un intervalle mobile d'observation t1 et le nombre de gouttes qu'il y aurait dû avoir pendant ledit intervalle t1 en cas d'une répartition uniforme des gouttes sur la durée totale initialement prévue pour l'infusion. Sur cette même échelle peuvent alors être indiqués les marges de tolérance positive et négative. Ces valeurs relatives peuvent être affichées en pourcentage, ce qui évite de se référer aux valeurs correspondant à un écart nul.

Afin de permettre un correction aisée d'écarts qui se produisent pendant la durée de perfusion, la première unité de calcul calcule en temps réel un nombre optimal de gouttes qui est défini comme suit:

$$\frac{(\Sigma \text{gouttes à passer})-(\Sigma \text{gouttes déjà passées})}{(\text{Durée totale prévue pour l'infusion})-(\text{Durée déjà écoulée de l'infusion})}$$

le tout multiplié par la durée t1 de l'intervalle d'observation mobile. Cette valeur optimale peut alors être affichée graphiquement sur ladite échelle linéaire. Si l'on veut corriger un écart, il suffit de varier le débit pour faire correspondre sur l'échelle linéaire un repère mobile correspondant au nombre de gouttes détectées à un repère mobile correspondant au nombre optimal de gouttes.

Le capteur raccordé à l'unité de surveillance autonome comprend de préférence une source de rayonnement infrarouge fonctionnant en mode pulsé et un récepteur optique activé de façon synchrone. L'émission/réception en mode pulsé permet d'améliorer le rapport signal/bruit (S/N), de rendre le système moins sensible à la lumière artificielle (50 ou 60 Hz) et de limiter en même temps la consommation d'énergie du capteur. En effet, au lieu de répartir l'énergie émise uniformément dans le temps, elle est concentrée dans des pics d'émission à amplitude élevée. De plus, le récepteur n'est activé qu'au moment où un signal lumineux est émis.

Le récepteur optique comprend avantageusement plusieurs photodiodes qui sont connectées électriquement en série et qui sont réparties dans un secteur angulaire en face de ladite source de rayonnement infrarouge. Il s'agit d'un récepteur bon marché qui permet de détecter non seulement les gouttes passant en une région proche de l'axe central de l'ampoule, mais aussi celles passant à une certaine distance de cet axe, lorsque l'ampoule compte-gouttes est inclinée. De plus, ce détecteur est très sensible à des variations de l'intensité lumineuse qui concernent une portion très faible de la surface totale photosensible.

Pour accoupler passagèrement l'interface d'entrée de l'unité de surveillance à ladite interface de sortie de la console de programmation on utilise avantageusement un opto-coupleur. Il s'agit d'un mode de couplage bon marché et fiable dans un milieu hospitalier de plus en plus pollué par des ondes électromagnétiques parasites.

Pour permettre un réglage initial aisé du débit de la perfusion, on ajoute de préférence dans l'unité de surveillance autonome des moyens de commutation pour commuter d'un mode de fonctionnement "surveillance/alarmes" dans un mode de fonctionnement "ajustage de débit". Dans ce dernier mode de fonctionnement les alarmes sonores sont hors service et une unité sonore produit avantageusement un signal acoustique pour chaque goutte détectée. L'infirmière dispose ainsi d'un instrument performant, entièrement préprogrammé, pour effectuer le réglage initial d'une perfusion par gravité. Des calculs du nombre de gouttes et des chronométrages manuels des gouttes ne sont plus requis. Il est par conséquent impossible de commettre une erreur en ce qui concerne le réglage du débit.

Des caractéristiques et avantages supplémentaires de la présente invention pourront être déduits de la description détaillée des Figures ci-jointes, qui représentent à titre d'exemple des exécutions préférées d'un dispositif selon l'invention. En particulier:

- la Figure 1 représente une vue d'ensemble d'un système de surveillance de perfusions selon l'invention, comprenant une console de programmation indépendante avec imprimante, reliée optionnellement à un ordinateur, et des unités de surveillance autonomes, munies chacune d'un capteur compte-gouttes;
- la Figure 2 représente le clavier de la console de programmation indépendante;
- la Figure 3 représente une vue d'une unité de surveillance autonome;
- la Figure 4 représente une vue du capteur compte gouttes;
- la Figure 5 est un schéma fonctionnel d'une unité de surveillance autonome;
- la Figure 6 est un schéma fonctionnel d'une unité de chronométrage et de comptage d'une unité de surveillance autonome;
- la Figure 7 est un schéma fonctionnel d'une console de programmation;
- la Figure 8 est un schéma fonctionnel d'un capteur compte-gouttes;
- la Figure 9 montre une configuration d'un écran de visualisation d'une unité de surveillance autonome.

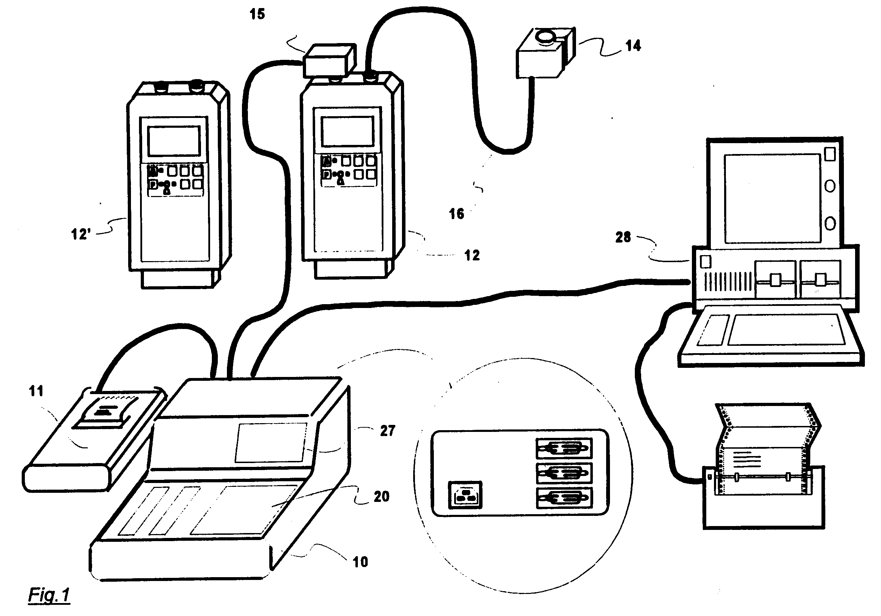

Sur la Figure 1 sont représentées les différentes composantes d'un dispositif selon l'invention. Il s'agit principalement d'une console de programmation 10, d'au moins une unité de surveillance autonome 12, 12' et d'un capteur compte-gouttes 14.

A chacune de ces unités de surveillance autonomes 12, 12' est connecté, à l'aide d'un câble 16, un capteur compte-gouttes 14 qui peut être monté sur une ampoule compte-gouttes (non représentée) tel que classiquement utilisée pour une perfusion par gravité. Un opto-coupleur 15 est utilisé pour accoupler passagèrement chacune des unités de surveillance autonomes 12, 12' à la console de programmation.

La console de programmation, l'unité de surveillance autonome et le capteur compte-gouttes seront maintenant étudiés en détail.

### 1) La console de programmation:

On se réfère simultanément à la Figure 1 et à la Figure 2, qui représentent le clavier d'une console de programmation selon l'invention.

Il est rappelé que la fonction de la console de programmation 10 est de programmer les unités de surveillance autonomes 12, 12', en d'autres mots, de charger les paramètres caractéristiques d'une perfusion déterminée - tels que le volume de perfusion, la durée de perfusion, le volume d'une goutte - dans les unités de surveillance autonomes. Pour permettre au personnel médical d'entrer ces paramètres sans problème et dans un environnement convivial dans la console de programmation 10, celle-ci comprend avantageusement un clavier 20 tel que représenté sur la Figure 2. Ce clavier 20 dispose pour chaque type de paramètres à entrer, d'un bloc distinct 21, 22, 23, 24 de touches. La référence 21 repère le bloc de touches utilisé pour entrer le volume de la perfusion. On voit qu'à chaque touche est affecté un volume habituellement utilisé pour une perfusion par gravité, par exemple: 1000 ml, 500 ml, 250 ml, 125 ml, 50 ml, 20 ml, 10 ml et 5 ml. La référence 22 repère un bloc analogue pour entrer la durée de la perfusion. Ce bloc 22 comprend des touches individuelles pour entrer les durées les plus habituelles d'une perfusion. La référence 23 repère un bloc analogue pour entrer des marges d'erreur, par exemple: 20%, 10%, 5% et 2%, en avance ou en retard par rapport au débit théorique. La référence 24 repère un bloc analogue pour entrer des paramètres de calibration du dispositif, à savoir soit le type de liquide de perfusion utilisé et le type d'ampoule compte-gouttes utilisée, soit directement le nombre de gouttes par ml.

Des touches de fonction 25 permettent d'ajouter ou de retrancher des valeurs pour chaque type de paramètre et de corriger les valeurs entrées, de confirmer la valeur d'un paramètre inhabituel ou d'une combinaison s'écartant d'une norme de sécurité, de choisir des combinaisons de paramètres ou de calibration (par exemple le nombre de gouttes par unité de volume). Enfin, une combinaison de deux touches 26 permet de valider les paramètres entrés et de les envoyer dans l'unité de surveillance autonome 12.

La console 10 comprend des moyens de visualisation 27, par exemple un écran à cristaux liquides. Cet écran 27 affiche les combinaisons de valeurs entrées, ainsi que des messages d'avertissement en cas d'anomalie de programmation. La console 10 peut être reliée à un ordinateur 28, par exemple à l'aide d'une liaison du type série, afin de communiquer et de recevoir des informations de prescription, ou des renseignements cliniques sur le patient (par exemple:

une insuffisance cardiaque, qui impose de limiter le débit et la marge d'erreur supérieure pour éviter une surcharge volumique pouvant induire un oedème pulmonaire).

A la console de commande 10 peut encore être reliée une imprimante 11 qui édite un récapitulatif des paramètres programmés. Ce récapitulatif, qui porte le numéro d'identification de l'unité de surveillance autonome 12 qui vient d'être programmée, sera glissé dans une fenêtre de visualisation sur l'unité de surveillance autonome 12, ceci afin d'éviter des permutations accidentelles entre des unités de surveillance autonomes 12, 12' programmées différemment.

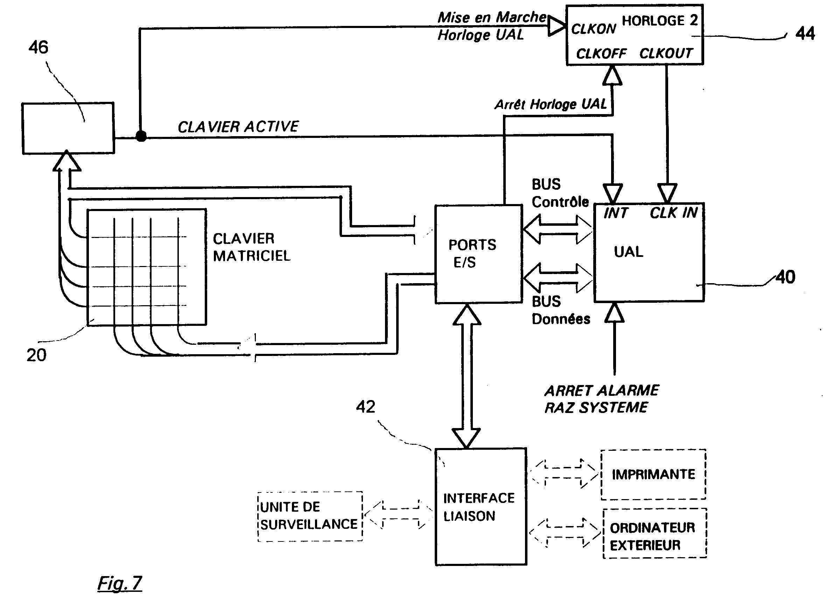

Le fonctionnement et la structure interne de la console de programmation 10 seront brièvement décrits à l'aide de la Figure 7. Chaque rectangle représente une unité fonctionnelle différente. Il va de soi que la réalisation pratique d'une telle unité fonctionnelle peut faire intervenir soit un ou plusieurs circuits électroniques conçus pour réaliser exclusivement la fonction décrite, soit un ou plusieurs circuits multifonctionnels qui réalisent aussi des fonctions additionnelles.

La console de programmation comprend principalement le clavier matriciel 20 (qui a été décrit en détail plus haut), une unité de calcul (unité arithmétique et logique 40), une horloge 44 associée à l'unité de calcul 40 et une interface d'entrée et de sortie 42. Les paramètres entrés par le clavier matriciel 20 sont transmis à l'unité de calcul 40 où ils sont préconditionnés avant d'être transmis par l'interface de liaison 42 à une unité de surveillance 12. Après programmation d'une unité de surveillance autonome, l'horloge 44 de l'unité de calcul 40 est arrêtée et l'unité de calcul est mise en mode "stand-by". Du moment qu'une des touches du clavier 20 est poussée, un opérateur "non-ou" 46 génère un signal qui redémarre l'horloge 44 et réactive l'unité de calcul 40. Ce mode de fonctionnement permet d'économiser de l'énergie électrique.

## 2) Les unités de surveillance autonomes.

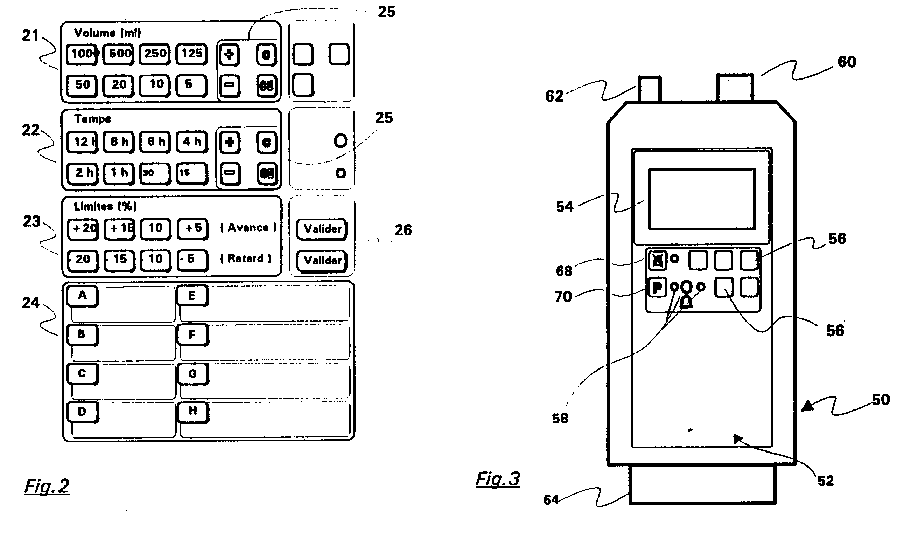

On se réfère d'abord à la Figure 3, qui représente l'aspect général extérieur d'une unité de surveillance autonome 12 selon l'invention. L'unité de surveillance autonome 12 est intégré dans un boîtier 50, de préférence un boîtier fermé hermétiquement pour faciliter la stérilisation de l'unité. Sur sa surface frontale 52 le boîtier 50 est muni d'un écran de visualisation LCD 54, de différents boutons commande 56 et de lampes témoins 58.

Ce boîtier 50 présente en outre une prise d'entrée 60 pour y connecter le câble de raccordement du capteur 14 et une fenêtre optique 62 pour y connecter l'opto-coupleur 15 relié électriquement à la console de programmation (c.f. Figure 1). Il sera remarqué que le boîtier 50 ne comprend pas de moyens pour entrer des paramètres de la perfusion. En effet, chaque unité de surveillance autonome 12 est programmée pour une perfusion déterminée et est affectée à celle-ci pendant toute sa durée. Un changement des paramètres programmés n'est en principe pas requis et devrait se faire, le cas échéant, en raccordant l'unité de surveillance autonome 12 de nouveau à la console de programmation.

La référence 64 repère une batterie d'accumulateurs qui est branchée dans le boîtier de façon à pouvoir être échangée en cours de fonctionnement. Une pile ou un accumulateur de secours, incorporé dans l'unité elle-même, assure l'alimentation lors de l'échange de la batterie d'accumulateurs 64 épuisée, afin qu'il n'y ait pas de discontinuité dans la surveillance. Alternativement ou additionnellement, le boîtier peut également être pourvu de connecteurs pour une batterie externe de manière à ce que l'on puisse enlever une batterie d'accumulateurs 64 sans causer une interruption de l'alimentation électrique. Un signal d'avertissement précède l'épuisement complet de la batterie interchangeable, d'une durée de trente minutes environ.

A l'intérieur du boîtier 50 est intégrée une unité d'alarme sonore. Un bouton 68 permet d'arrêter l'alarme sonore, qui se met cependant de nouveau automatiquement en marche si le défaut détecté subsiste.

Une touche repérée par la référence 70 permet de supprimer la surveillance pendant un temps fixé pour permettre une intervention sur la perfusion.

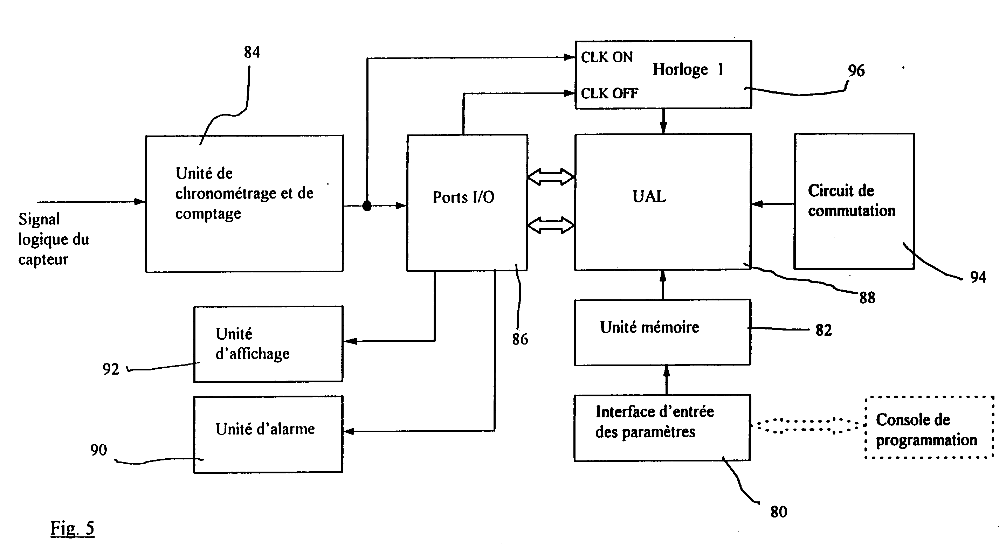

Le fonctionnement et la structure interne de l'unité de surveillance autonome seront brièvement décrits à l'aide de la Figure 5. Chaque rectangle représente une unité fonctionnelle différente. Il va de soi que la réalisation pratique d'une telle unité fonctionnelle peut faire intervenir soit un ou plusieurs circuits électroniques conçus pour réaliser exclusivement la fonction décrite, soit un ou plusieurs circuits multifonctionnels qui réalisent aussi des fonctions additionnelles.

Les paramètres de la perfusion sont entrés par une interface d'entrée des paramètres 80, dont fait partie la fenêtre optique 60. Ces paramètres de la perfusion sont mémorisés dans une unité de mémoire 82, qui est raccordée à une unité de calcul 88. L'interface d'entrée 80 renvoie à la console de programmation 10 un protocole de réception.

Un signal logique 83 provenant du capteur compte-gouttes 14 est reçu par une unité de chronométrage et de comptage 84. Cette dernière chronomètre l'intervalle de temps qui sépare deux gouttes, ou fournit un signal de dépassement de temps en l'absence de gouttes au-delà d'un délai limite. Dans l'éventualité du passage d'une goutte, celle-ci déclenche un signal spécifique. Par l'intermédiaire des ports I/O 86, l'unité de calcul 88 est donc informée du temps séparant deux gouttes ou du retard excessif d'apparition d'une goutte.

Un algorithme de calcul, géré par l'unité de calcul 88, compare les données reçues de l'unité de chronométrage et de comptage 84 aux valeurs stockées dans l'unité mémoire 82 et déclenche une alarme sonore et/ou visuelle sur une unité d'alarme 90, par exemple si l'intervalle de temps mesuré entre plusieurs gouttes consécutives dépasse des marges de tolérance préprogrammées. L'unité de calcul 88 gère aussi une unité d'affichage 92, permettant aussi bien l'affichage de paramètres mesurés que de paramètres programmés.

La référence 96 indique un oscillateur haute fréquence synchronisant l'unité de calcul 88. Entre des événements successifs (passage d'une goutte, dépassement du temps limite entre deux gouttes ou défaut du capteur), une fois terminé le traitement de l'information, la consommation électrique de l'unité de surveillance est réduite par une mise hors service de l'unité de calcul 88 par arrêt de l'oscillateur 96.

Pour permettre un réglage initial aisé du débit de la perfusion, on ajoute de préférence dans l'unité de surveillance autonome un circuit de commutation 94 pour commuter d'un mode de fonctionnement "surveillance/alarmes" dans un mode de fonctionnement "ajustage de débit". Dans ce dernier mode de fonctionnement les alarmes sonores sont hors service et une unité sonore produit avantageusement un signal acoustique pour chaque goutte détectée. L'infirmière dispose ainsi d'un instrument performant, entièrement préprogrammé, pour effectuer le réglage initial d'une perfusion par gravité. Des calculs du nombre de gouttes et des chronométrages manuels des gouttes ne sont plus requis. Il est par conséquent impossible de commettre une erreur en ce qui concerne le réglage du débit.

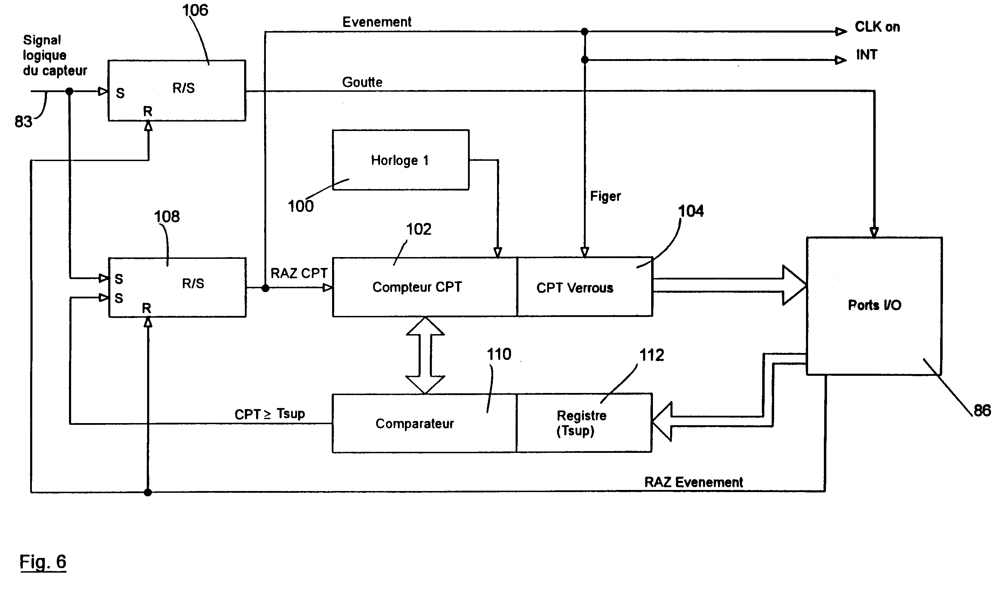

La description de l'unité de chronométrage et de comptage des gouttes s'effectue en se référant à la figure 6.

Les impulsions d'une horloge 100 à basse fréquence sont décomptées par des circuits diviseurs-compteurs 102. Pour une fréquence de 512 Hz, un comptage sur 16 bits permet d'atteindre un temps de plus d'une minute entre deux gouttes, ce qui équivaut à environ trois à quatre fois le temps limite pour un débit très faible (250 ml en 24 heures). La valeur des circuits diviseurs-compteurs 102 est en même temps passée à des circuits verrous (data-latch) 104.

Le signal logique 83 provenant du capteur 14 et indiquant le passage d'une goutte provoque une transition (low → high) à la sortie de deux bascules-SR 106 et 108. La transition de 106 indique à l'unité de calcul 88 qu'il y a eu passage d'une goutte, tandis que la transition de 108 indique seulement qu'il y a eu un événement (passage d'une goutte, dépassement du temps limite entre deux gouttes ou défaut du capteur). Le signal d'événement redémarre l'oscillateur 96 de l'unité de calcul, provoque une interruption de l'unité de calcul 88. Parallèlement, le signal d'événement fige le décompte des impulsions sur les circuits verrous 104 et remet les compteurs d'impulsions 102 à zéro. La valeur des compteurs 102 conservée sur les circuits verrous 104 sert à déterminer le temps écoulé depuis le dernier signal de goutte. En présence du signal de goutte fourni par la bascule-SR 106, cette valeur peut être cumulée par l'unité de calcul 88 sur une variable sur quatre octets (26 bits significatifs pour 24 heures), ce qui permet de chronométrer le temps total écoulé depuis le début, sans nécessiter de compteur de temps supplémentaire. Le nombre de gouttes écoulées peut être accumulé sur une variable sur deux octets (15 bits significatifs pour un volume de 1600 ml). Après le traitement de ces données par l'unité de calcul, celle-ci remet les deux bascules-SR 106 et 108 à zéro, ce qui redémarre également le décompte des impulsions sur les circuits verrous 104. Ensuite l'unité de calcul 88 est mise en état d'attente, à faible consommation, par l'arrêt de son oscillateur 96.

En l'absence de signal d'une goutte au capteur, un comparateur 110 compare en permanence la valeur des compteurs 102 en permanence à un intervalle de temps limite entre deux gouttes. Ce dernier, qui est stocké dans un registre 112 de l'unité mémoire 82, a été fixé par la programmation de l'unité de surveillance en fonction du volume et de la durée de la perfusion. . Si la valeur des compteurs 102 dépasse ce temps limite, le comparateur 110 provoque une transition (low → high) à la sortie de la bascule-SR 108 sans provoquer une transition à la sortie de la bascule-SR 106. Cet état logique sera interprété par l'algorithme de l'unité de calcul 88 comme situation nécessitant un déclenchement d'une alarme.

L'algorithme de calcul de l'unité de surveillance autonome est avantageusement conçu pour déclencher différents niveaux d'alarmes, par exemple:

- absence d'un signal de goutte pendant un intervalle de temps supérieur à un intervalle de temps autorisé (fixé par exemple en tenant compte d'un intervalle moyen calculé et d'un écart relatif extrême fixe); ce qui peut correspondre soit à une absence d'écoulement, soit à un écoulement continu par l'orifice compte-gouttes: alarme prioritaire absolue.
- débit hors des limites fixées pour plusieurs gouttes consécutives: avertissement par un signal bref lors de la détection de la goutte.
- débit instantané hors des limites extrêmes (p. ex.: écart absolu supérieur à 25%) entre deux gouttes: avertissement.
- débit instantané hors des limites extrêmes, sur plusieurs gouttes consécutives: alarme continue.
- arrêt momentané par l'opérateur ( par exemple, pour intervention sur la perfusion ou soins): signalisation par signal spécifique à intervalle de temps fixe, de la mise en veille du boîtier de surveillance, et signalisation du passage de chaque goutte.
- épuisement de la batterie: alarme prioritaire pouvant être momentanément désactivée, pour une période correspondant à une fraction de la durée de réserve (batterie interne de secours), puis alarme non désactivable en cas de défaut de remplacement de la batterie.

L'algorithme de calcul permet aussi de calculer les indicateurs suivants qui renseignent en continu sur l'état de la perfusion:

- Fraction du volume total perfusé:
  c'est le rapport du nombre de gouttes écoulées sur celui des gouttes du volume total à perfuser; il s'agit d'un indicateur de l'avancement de la perfusion, plus précis que les graduations sur les récipients (de préférence affichage en %).
- Débit optimal pour maintenir le temps total égal au temps prévu au départ:
  c'est la différence entre le nombre de gouttes correspondant au volume total de la perfusion et le total des gouttes déjà écoulées, divisée par la différence entre la durée totale prévue et le temps passé depuis le départ (de préférence affichage en % relatif).;
- Débit instantané:
  c'est une grandeur inversément proportionnelle au temps entre deux gouttes consécutives;
- Débit moyen:
  c'est le rapport entre le nombre de gouttes perfusées et le temps écoulé depuis le départ.

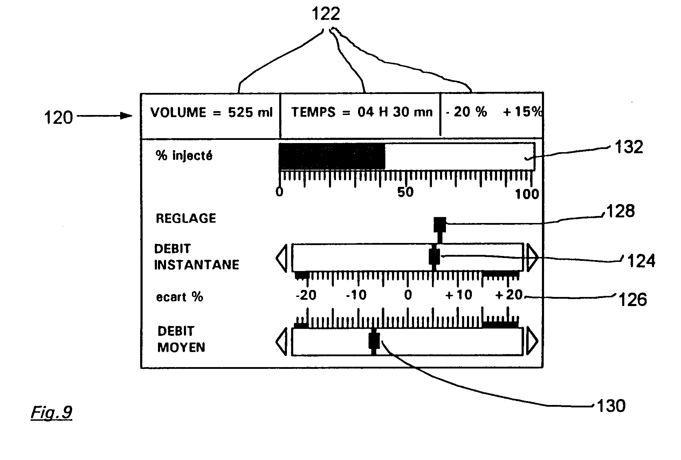

La Figure 9 représente un mode d'affichage préférentiel de ces indicateurs. Tout en haut de l'affichage il y a une ligne d'état 120 indiquant les paramètres préprogrammés de la perfusion 122. En dessous, un premier curseur graphique 124, pseudo-analogique, se déplace sur une échelle 126 graduée en pourcentage d'écarts par rapport aux valeurs idéales initialement entrées ou calculées et représente une moyenne mobile du débit (cette moyenne mobile est par exemple calculée sur les huit dernières gouttes). Un deuxième curseur 128 mobile représente le débit optimal calculé pour respecter la durée initialement prévue pour la perfusion. Un troisième curseur 130 indique le débit moyen depuis le début de la perfusion. Finalement, un troisième affichage 132 représente la fraction du volume total perfusé.

Une unité de surveillance autonome 12 peut encore comprendre comme équipement supplémentaire un émetteur ou un émetteur-récepteur qui envoie soit des données et/ou des alarmes vers une centrale de surveillance (le cas échéant, éventuellement par l'intermédiaire d'un relais répétiteur). L'émetteur-récepteur permet une interrogation à distance de chaque unité de surveillance autonome, par envoi d'une séquence codée d'identification correspondant à l'unité de surveillance autonome qu'on veut interroger. Une surveillance à distance de tous les indicateurs de l'avancement de l'infusion est dès lors possible.

### 3) Le Capteur compte-gouttes

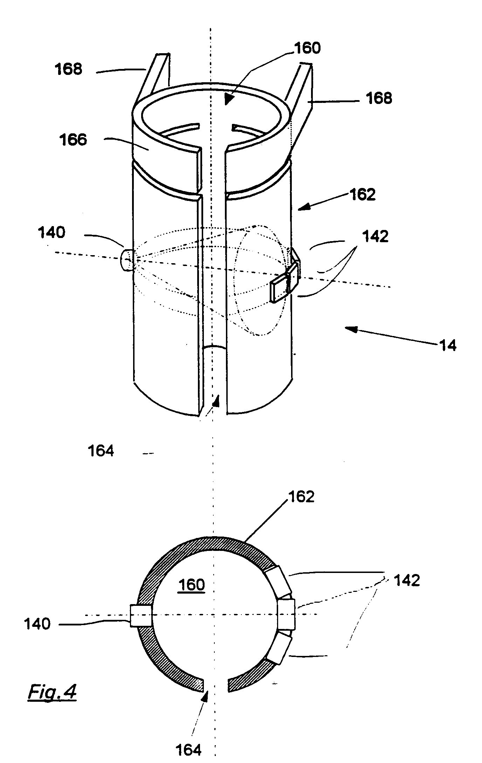

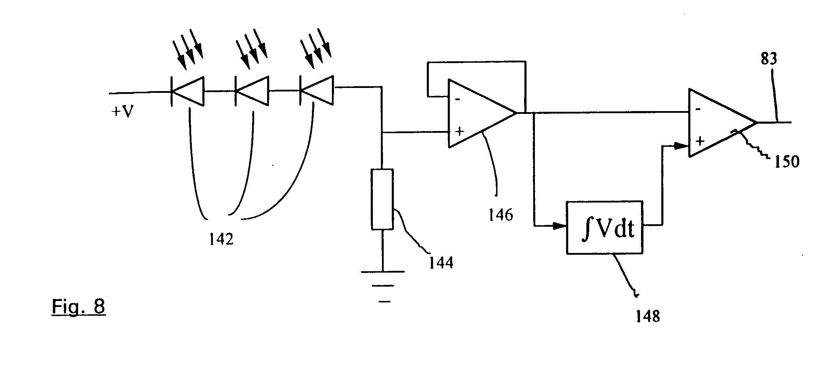

En ce qui concerne la description du capteur, on se réfère aux Figures 4 et 8. Le capteur 14 relié à une unité de surveillance autonome 12 est un capteur électro-optique, du type à barrière optique, utilisant une source 140 et un récepteur 142 travaillant de préférence dans le proche infrarouge (800 à 950 nanomètres). Cette plage de travail présente en effet des avantages techniques notables, tels que: faible temps de réponse des émetteurs et des détecteurs; absence d'effets de mémoire des récepteurs; faible sensibilité aux éclairements parasites.

L'émetteur 140 et le récepteur 142 travaillent en mode pulsé synchrone, ce qui présente notamment les avantages suivants:

- insensibilité élevée aux éclairements parasites (50 Hz ou 60 Hz), qui sont très intenses dans ce domaine spectral lors de l'utilisation de sources lumineuses thermiques à haute température, du type lampes à incandescence "halogènes";
- sensibilité élevée liée à une faible consommation, l'émission se faisant à des intervalles fixes, pendant une durée très brève (rapport cyclique faible); en d'autres mots les pics d'émission de l'émetteur peuvent être très puissants alors que sa consommation moyenne reste faible.

Il sera apprécié que le capteur proposé est conçu de façon à détecter non seulement les gouttes passant en une région proche de l'axe central de l'ampoule compte-gouttes, mais aussi celles passant à distance de cet axe, par exemple lorsque l'ampoule est inclinée ou subit des vibrations ou des chocs. A cette fin le récepteur comprend plusieurs photodiodes 142 (par exemple trois photodiodes) agencées dans un secteur angulaire en face de la source 140. Chacune de ces photodiodes est en mesure de limiter un courant passant dans une résistance de polarisation 144 placée dans l'entrée d'un amplificateur adaptateur d'impédance 146, au cas où elle reçoit une quantité de lumière diminuée lors du passage de la goutte . Contrairement aux systèmes à faisceau étroit, où la goutte doit intercepter une portion élevée du faisceau lumineux pour déclencher le signal de détection, il suffit ici d'une variation modérée du flux global pour détecter une goutte. Le signal de l'amplificateur adaptateur d'impédance 146 est alors intégré par un amplificateur intégrateur 148 afin de fournir une valeur moyenne, amplifiée par un facteur ajustable. Un comparateur 150 compare cette valeur moyenne au signal non intégré et fournit ainsi le signal logique 83 du capteur 14.

L'ampoule compte-gouttes est avantageusement introduite dans un canal cylindrique de mesure 160, aménagé dans un bloc support 162. Ce canal de mesure 160 a de préférence une surface interne réfléchissante au niveau de la barrière optique, afin de provoquer des réflexions multiples de la lumière émise par la source. Les photodiodes 142 du récepteur détectent alors la perturbation qu'entraîne le passage de la goutte dans ce système de réflexions multiples à l'intérieur du canal de mesure 160. Il sera apprécié qu'avec le système de détection proposé il est quasi impossible de manquer une goutte.

Sur la Figure 4 on voit que le canal de mesure 160 est accessible par une fente latérale 164 aménagée dans le bloc de support 162 le long d'une génératrice du canal cylindrique. Cette fente 164 a une largeur permettant le passage du tube souple amenant le liquide de l'ampoule vers le cathéter de perfusion (tube communément dénommé "tubulure"). Ceci permet d'introduire facilement l'ampoule compte-gouttes dans le canal de mesure. La partie supérieure 166 de ce bloc support 162 comprend de préférence deux branches flexibles qui peuvent être écartées et qui se resserrent après relâchement autour de la partie supérieure de l'ampoule pour la maintenir en place. Deux leviers 168, agencés du côté opposé de la fente, permettent lorsqu'ils sont pincés d'écarter les deux branches afin de positionner le bloc support sur l'ampoule. Les éléments optiques et électroniques sont agencés latéralement, par exemple dans un ou deux boîtiers attenant au bloc de support 162.

**Revendications**

1.  Dispositif de surveillance de perfusions, comprenant:

    un capteur (14) apte à être associé à une ampoule compte-gouttes pour détecter la présence, respectivement l'absence d'une goutte;
    une unité de chronométrage et de comptage (84) des gouttes détectées par ledit capteur (14);
    une unité d'entrée de données (20) apte à entrer des paramètres caractéristiques de l'infusion;
    une unité mémoire (82) pour stocker des paramètres caractéristiques de l'infusion;
    une unité d'affichage (54);
    une unité d'alarme acoustique et/ou visuelle (90); et
    une première unité de calcul (88) apte à utiliser un algorithme de calcul, faisant intervenir comme paramètre un signal de l'unité de chronométrage et de comptage, ainsi que les paramètres caractéristiques de l'infusion stockés dans l'unité de mémoire, pour déclencher une alarme acoustique et/ou visuelle,
    **caractérisé**
    en ce que ladite unité d'entrée de données (20) est intégrée dans une console de programmation autonome et indépendante (10),
    en ce que cette console de programmation (10) comprend une deuxième unité de calcul (40) et une interface de sortie (42), ladite deuxième unité de calcul (40) étant apte à préconditionner les paramètres caractéristiques de l'infusion entrés sur l'unité d'entrée de données des paramètres (20), et ladite interface de sortie (42) étant apte à transformer ces paramètres préconditionnés en signaux transmissibles à distance,
    en ce que ladite unité de chronométrage et de comptage (84), ladite unité mémoire (82), ladite unité d'affichage (92), ladite unité d'alarme acoustique et/ou visuelle (90) et ladite première unité de calcul (88) sont intégrées dans un premier boîtier ensemble avec une source autonome d'alimentation électrique (64) qui les alimente; cet ensemble constituant une unité de surveillance autonome et indépendante (12) raccordée en permanence audit capteur (14) associé à ladite ampoule compte-gouttes pendant l'infusion; et
    en ce que cette unité de surveillance autonome et indépendante (12) comprend une interface d'entrée (80) qui est apte à reconstituer lesdits paramètres préconditionnés à partir desdits signaux générés par ladite interface de sortie (42), de façon à ce que lesdits paramètres préconditionnés puissent être chargés dans ladite unité mémoire (82) pour utilisation par ladite première unité de calcul (88).

2.  Dispositif selon la revendication 1, caractérisé en ce que ladite unité d'entrée de données (20) comprend des moyens (21) pour entrer un volume de l'infusion et des moyens (22) pour entrer une durée de l'infusion.

3.  Dispositif selon la revendication 1 ou 2, caractérisé en ce que ladite unité d'entrée de données (20) comprend en outre des moyens (23) pour entrer une marge de tolérance applicable au nombre de gouttes détectées dans un intervalle de temps, cette marge de tolérance intervenant dans ledit algorithme de calcul pour retarder le déclenchement d'une alarme.

4.  Dispositif selon la revendication 3, caractérisé en ce que lesdits moyens pour entrer une marge de tolérance du débit d'infusion comprennent

    des moyens pour entrer une première marge de tolérance applicable au nombre de gouttes détectées dans un intervalle de temps, dans le cas où ce nombre est inférieur à une valeur moyenne calculée pour le même intervalle de temps; et

des moyens pour entrer une deuxième marge de tolérance applicable au nombre de gouttes détectées dans un intervalle de temps, dans le cas où ce nombre est supérieur à une valeur moyenne calculée pour le même intervalle de temps.

5. Dispositif selon l'une quelconque des revendications 1 à 4, caractérisé en ce que ladite unité d'entrée de données comprend en outre des moyens (24) pour entrer des paramètres servant à la calibration.

6. Dispositif selon l'une quelconque des revendications 1 à 5, caractérisé en ce que la console de programmation comprend comme unité d'entrée de données un clavier à touches dont chaque touche est dédiée à une valeur précise d'un paramètre caractéristique de l'infusion.

7. Dispositif selon l'une quelconque des revendications 1 à 6, caractérisé en ce que ladite unité d'affichage (54) comprend des moyens pour afficher des paramètres caractéristiques de l'infusion déduits des paramètres chargés dans ladite unité de stockage de l'unité de surveillance autonome.

8. Dispositif selon la revendication 7, caractérisé en ce que les paramètres caractéristiques affichés comprennent le volume de l'infusion, la durée de l'infusion et les marges de tolérance.

9. Dispositif selon l'une quelconque des revendications 1 à 8, caractérisé en ce que ladite unité d'affichage (54) comprend des moyens pour afficher graphiquement sur une échelle linéaire l'écart positif ou négatif entre le nombre de gouttes détectées pendant un intervalle mobile d'observation t1 et le nombre de gouttes qu'il aurait dû y avoir pendant ledit intervalle t1 en cas d'une répartition uniforme des gouttes sur la durée totale initialement prévue pour l'infusion.

10. Dispositif selon la revendication 9, caractérisé en ce que ladite première unité de calcul est apte à calculer en continu un nombre optimal de gouttes qui est affiché sur ladite échelle linéaire sous forme d'un curseur (128) indiquant l'écart relatif dudit nombre optimal de gouttes par rapport au nombre correspondant à l'écart nul et qui est défini comme suit:

$$\frac{(\Sigma \text{gouttes à passer})-(\Sigma \text{gouttes déjà passées})}{(\text{Durée totale prévue pour l'infusion})-(\text{Durée déjà écoulée de l'infusion})}$$

multiplié par la durée t1 de l'intervalle d'observation mobile.

11. Dispositif selon l'une quelconque des revendications 1 à 10, caractérisé en ce que ledit capteur (14) comprend une source de rayonnement infrarouge (140) fonctionnant en mode pulsé et un récepteur optique activé de façon synchrone.

12. Dispositif selon la revendication 11, caractérisé en ce que ledit récepteur optique comprend plusieurs photodiodes (142) qui sont connectées électriquement en série et qui sont réparties dans un secteur angulaire en face de ladite source de rayonnement infrarouge.

13. Dispositif selon la revendication 11 ou 12, caractérisé en ce que ledit capteur (14) comprend

un amplificateur intégrateur (148) pour intégrer le signal dudit récepteur optique et fournir comme signal de sortie une valeur moyenne amplifiée par un facteur ajustable; et

un comparateur (150) pour comparer ledit signal de sortie au signal non intégré dudit récepteur et fournir un signal logique (83) comme signal d'entrée pour l'unité de chronométrage et de comptage (84).

14. Dispositif selon l'une quelconque des revendications 1 à 13, caractérisé en ce que pour accoupler passagèrement l'interface d'entrée de l'unité de surveillance à ladite interface de sortie de la console de programmation on utilise un opto-coupleur (15).

15. Dispositif selon l'une quelconque des revendications 1 à 14, caractérisé en ce que ladite unité de surveillance autonome (12) comprend en outre

des moyens de commutation (94) pour commuter d'un mode de fonctionnement surveillance/alarmes dans un mode de fonctionnement ajustage de débit, dans lequel les alarmes sonores sont hors service; et

une unité sonore apte à produire un signal acoustique pour chaque goutte détectée dans ledit mode "ajustage de débit".

16. Dispositif selon l'une quelconque des revendications 1 à 15, caractérisé en ce que ladite unité de surveillance autonome (12) comprend en outre des moyens pour mettre hors service ladite première unité calcul (88) par arrêt de son oscillateur (96) entre des événements successifs, où un événement est assimilé au passage d'une goutte au dépassement du temps limite entre deux gouttes ou au défaut du capteur.

17. Dispositif selon la revendication 16, caractérisé en ce que ladite unité de chronométrage et de comptage (84) comprend

des premiers moyens logiques (108) pour générer une transition d'un premier signal logique lors d'un événement de premier type, correspondant soit au passage d'une goutte, soit au dépassement du temps limite entre deux gouttes ou soit à un défaut du capteur, lesdits premiers moyens logiques redémarrant l'oscillateur (96) de l'unité de calcul (88) lors d'un événement de premier type;
des deuxièmes moyens logiques (106) pour générer une transition d'un deuxième signal logique exclusivement lors du passage d'une goutte, lesdits deuxièmes moyens logiques (106) déclenchant dans l'unité de calcul (88) le déroulement d'un algorithme de calcul lors du passage d'une goutte; et
des moyens pour générer une alarme lorsqu'il y a transition dudit premier signal logique et non-transition dudit deuxième signal logique.

18. Dispositif selon la revendication 16 ou 17, caractérisé en ce que ladite unité de chronométrage et de comptage (84) comprend en outre

un compteur d'impulsions (102) pour compter les impulsions d'une horloge basse fréquence (100);
un compteur-verrous (104) associé audit compteur d'impulsions (102) et connecté à l'unité de calcul (88) de façon à être interrogeable par cette dernière;
lesdits premiers moyens logiques étant connectés audit compteur d'impulsions (102) et audit compteur-verrous (104) de façon à figer le décompte des impulsions dans le compteur-verrous (104) et à remettre les compteurs d'impulsions (102) à zéro lors d'une transition dudit premier signal logique.

19. Dispositif selon la revendication 18, caractérisé en ce que ladite unité de chronométrage et de comptage (84) comprend en outre

un comparateur (110) comparant en permanence la valeur du compteur d'impulsions (102) à un intervalle de temps limite prédéterminé,
ledit comparateur (110) provoquant une transition dudit premier signal logique en cas de dépassement dudit intervalle de temps limite prédéterminé.

## Claims

1. Infusion monitoring device, comprising:

a sensor (14) suitable for connection to a drop counter ampoule, to detect the presence or absence respectively of a drop;
a chronometry and counter unit (84) for the drops detected by the sensor (14);
a data input unit (20) suitable for entering the characteristic parameters of the infusion;
a memory unit (82) for storing the characteristic parameters of the infusion;
a display unit (54);
an audible and/or visual alarm (90); and
a first calculation unit (88) capable of using a calculation algorithm, using as parameters a signal from the chronometry and counting unit, as well the characteristic parameters of the infusion which are stored in the memory unit, to actuate an audible and/or visual alarm,
**characterised in that:**
the data input unit (20) is integrated into an autonomous and independent programming console (10);
this programming console (10) comprises a second calculation unit (40) and an output interface (42), the second calculation unit (40) being capable of pre-processing the characteristic parameters of the infusion which are entered in the parameter data input unit (20) and the output interface (42) being capable of transforming these pre-processed parameters into remote transmittable signals;

the chronometry and counting unit (84), the memory unit (82), the display unit (92), the audible and/or visual alarm unit (90) and the first calculation unit (88) are integrated into a first assembly housing with an autonomous electric power supply (64); this assembly forming an autonomous and independent monitor unit (12) permanently connected to the sensor (14) associated with the drop counter ampoule during the infusion; and this autonomous and independent monitoring unit (12) comprises an input interface (80) which is capable of reconstituting the pre-processed parameters on the basis of the signals generated by the output interface (42), in such a way that the pre-processed parameters can be loaded into the memory unit (82) for use by the first calculation unit (88).

2. Device according to Claim 1, characterised in that the data input unit (20) comprises means (21) to input the volume of the infusion and means (22) to input a duration for the infusion.

3. Device according to Claims 1 or 2, characterised in that the data input unit (20) further comprises means (23) for entering a tolerance margin applicable to the number of drops detected in an interval of time, this tolerance margin taking effect in the calculation algorithm to delay the actuation of an alarm.

4. Device according to Claim 3, characterised in that the means for entering a tolerance margin for the rate of the infusion comprise:

means to enter a first tolerance margin applicable to the number of drops detected in an interval of time, in the situation in which this number is lower than a mean value calculated for the same interval of time; and

means for entering a second tolerance margin applicable to the number of drops detected in an interval of time, in the situation in which this number is greater than a mean value calculated for the same interval of time.

5. Device according to any of Claims 1 to 4, characterised in that the data input unit further comprises means (24) to enter the parameters which serve to carry out calibration.

6. Device according to any of Claims 1 to 5, characterised in that the programming console comprises, as a data input unit, a keyboard, each key of which is allocated to a precise value of a characteristic parameter of the infusion.

7. Device according to any of Claims 1 to 6, characterised in that the display unit (54) comprises means to display the characteristic parameters of the infusion, derived from the parameters entered in the memory unit of the autonomous monitoring unit.

8. Device according to Claim 7, characterised in that the characteristic parameters displayed comprise the volume of the infusion, the duration of the infusion, and the tolerance margins.

9. Device according to any of Claims 1 to 8, characterised in that the display unit (54) comprises means to display in graphic format, on a linear scale, the positive or negative discrepancy between the number of drops detected during a changeable period of observation t1 and the number of drops which should have been present during this period of time t1, in the event of a uniform distribution of the drops over the total period of time originally planned for the infusion.

10. Device according to Claim 9, characterised in that the first calculation unit is capable of calculating, on a continuous basis, an optimum number of drops, which is displayed on the linear scale in the form of a cursor (128), indicating the relative discrepancy between the optimum number of drops in relation to the number which corresponds to the zero discrepancy, and which is defined as follows:

$$\frac{(\Sigma \text{ drops to pass}) - (\Sigma \text{ drops already passed})}{(\text{Total duration planned for the infusion}) - (\text{Time already elapsed of the infusion})}$$

multiplied by the duration t1 of the changeable observation period.

11. Device according to any of Claims 1 to 10, characterised in that the sensor (14) comprises an infrared radiation source (140), functioning in pulsed mode, and an optical receiver activated in a synchronous manner.

12. Device according to Claim 11, characterised in that the optical receiver comprises several photodiodes (142) which are electrically connected in series and which are distributed in an angular sector opposite to the source of infrared radiation.

13. Device according to Claim 11 or 12, characterised in that the sensor (14) comprises:

   an integrator amplifier (148) to integrate the signal from the optical receiver and to supply, as an output signal, a mean value amplified by an adjustable factor; and
   a comparator (150) to compare the output signal with the non-integrated signal of the receiver and to supply a logic signal (83) as an input signal for the chronometry and counting unit (84).

14. Device according to any of Claims 1 to 13, characterised in that, in order to link temporarily the input interface of the monitoring unit with the output interface of the programming console, an opto-coupler (15) is used.

15. Device according to any of Claims 1 to 14, characterised in that the autonomous monitoring device (12) further comprises :

   switching means (94) to switch from a monitoring/alarm function mode to a rate adjustment function mode, in which the audible alarms are turned off; and
   a sound unit capable of producing an audible signal for each drop detected in the "rate adjustment" mode.

16. Device according to any of Claims 1 to 15, characterised in that the autonomous monitoring unit (12) further comprises means to turn off the first calculation unit (88), by stopping its oscillator element (96) between successive events, in which situation an event is interpreted as the passage of a drop, the exceeding of the limit time between two drops, or the failure of the sensor.

17. Device according to Claim 16, characterised in that the chronometry and counting unit (84) comprises:

   first logic means (108) to govern the transition of a first logic signal at the occurrence of an event of the first type, corresponding either to the passage of a drop, or to the exceeding of a limit time between two drops, or to the failure of the sensor, the first logic means restarting the oscillator element (96) of the calculation unit (88) at the occurrence of an event of the first type;
   second logic means (106) to govern the transition of a second logic signal exclusively at the passage of a drop, the second logic means (106) triggering in the calculation unit (88) the start of a calculation algorithm during the passage of a drop; and
   means for generating an alarm whenever there is a transition of the first logic signal and the non-transition of the second logic signal.

18. Device according to Claim 16 or 17, characterised in that the chronometry and counting unit (84) further comprises :

   a pulse counter (102) to count the pulses of a low-frequency clock (100);
   a latch counter (104) linked to the pulse counter (102) and connected to the calculation unit (88) in such a way as to be capable of being interrogated by the latter;
   the first logic means being connected to the pulse counter (102) and the latch counter (104) in such a way as to freeze the counting of the pulses in the latch counter (104) and to reset the pulse counters (102) to zero in the event of a transition of the first logic signal.

19. Device according to Claim 18, characterised in that the chronometry and counting unit (84) further comprises :

   a comparator (110) which continuously compares the value of the pulse counter (102) at a predetermined limit time interval;
   the comparator (110) incurring a transition of the first logic signal in the event of a predetermined limit time interval is being exceeded.

**Patentansprüche**

1. Vorrichtung zur Überwachung von Infusionen, mit

   einem Sensor (14), zur Anbringung an eine Tropfkammer, um den Durchlauf beziehungsweise das Ausbleiben eines Tropfens zu erfassen;
   einer Erfassung- und Zähleinheit (84) für die von diesem Sensor (14) erfassten Tropfen;
   einer Dateneingabeneinheit (20), zur Eingabe der spezifischen Parameter einer Infusion;
   einer Speichereinheit (82) zur Speicherung spezifischer Parameter der Infusion;
   einer Anzeigeeinheit (54);
   einer akustischen und/oder visuellen Alarmeinheit (90); sowie
   einer ersten Recheneinheit (88), die sich für die Verwendung eines Rechenalgorithmus eignet, der als Parameter ein Signal der Einheit zur zeitlichen Erfassung und Zählung sowie die in der Speichereinheit gespeicherten spezifischen Parameter der Infusion berücksichtigt, um einen akustischen und/oder visuellen Alarm auszulösen,
   **dadurch gekennzeichnet, daß**
   die Einheit zur Dateneingabe (20) in eine autonome und unabhängige Programmierkonsole (10) integriert ist,
   diese Programmierkonsole (10) eine zweite Recheneinheit (40) und eine Ausgangsschnittstelle (42) umfaßt, wobei diese zweite Recheneinheit (40) dazu geeignet ist, die auf der Einheit zur Eingabe der Parameterdaten (20) eingegebenen spezifischen Infusionsparameter vorab aufzubereiten, und diese Ausgangsschnittstelle (42) dazu geeignet ist, diese vorab aufbereiteten Parameter in fernübertragbare Signale umzuwandeln,
   die Erfassung- und Zähleinheit (84), die Speichereinheit (82), die Anzeigeeinheit (92), die akustische und/oder visuelle Alarmeinheit (90) sowie die erste Recheneinheit (88) gemeinsam mit einer autonomen Stromquelle (64), die sie versorgt, in ein erstes Gehäuse integriert sind, wobei diese Gesamtheit eine autonome und unabhängige Überwachungseinheit (12) darstellt, die während der Infusion ständig an den an der Tropfkammer angebrachten Sensor (14) angeschlossen ist; und
   diese autonome und unabhängige Überwachungseinheit (12) eine Eingangsschnittstelle (80) umfaßt, die dazu geeignet ist, ausgehend von den von der Ausgangsschnittstelle (42) erzeugten Signalen die vorab aufbereiteten Parameter wiederherzustellen, so daß diese vorab aufbereiteten Parameter für die Benutzung durch die erste Recheneinheit (88) in die Speichereinheit (82) einladbar sind.

2. Vorrichtung nach Anspruch 1, dadurch gekennzeichnet, daß die Dateneingabeeinheit (20) Mittel (21), um einen Infusionsinhalt einzugeben, sowie Mittel (22), um eine Infusionsdauer einzugeben, umfaßt.

3. Vorrichtung nach Anspruch 1 oder 2, dadurch gekennzeichnet, daß die Dateneingabeeinheit (20) außerdem Mittel (23) umfaßt, um einen Toleranzbereich einzugeben, der auf die Anzahl in einem Zeitraum erfasster Tropfen anwendbar ist, wobei dieser Toleranzbereich in dem Rechenalgorithmus berücksichtigt wird, um die Auslösung eines Alarms zu verzögern.

4. Vorrichtung nach Anspruch 3, dadurch gekennzeichnet, daß diese Mittel, um einen Toleranzbereich der Infusionsmenge einzugeben, Mittel umfassen

   um einen ersten Toleranzbereich einzugeben, der auf die in einem Zeitraum erfasste Anzahl von Tropfen anwendbar ist, falls diese Anzahl unter einem für den gleichen Zeitraum berechneten Mittelwert liegt; und
   um einen zweiten Toleranzbereich einzugeben, der auf die in einem Zeitraum erfasste Anzahl von Tropfen anwendbar ist, falls diese Anzahl über einem für den gleichen Zeitraum berechneten Mittelwert liegt.

5. Vorrichtung nach irgendeinem der Ansprüche 1 bis 4, dadurch gekennzeichnet, daß die Dateneingabeeinheit außerdem Mittel (24) umfaßt, um zur Kalibrierung dienende Parameter einzugeben.

6. Vorrichtung nach irgendeinem der Ansprüche 1 bis 5, dadurch gekennzeichnet, daß die Programmierungskonsole als Dateneingabeeinheit ein Tastenfeld umfaßt, dessen einzelne Tasten jeweils einem genauen Wert eines spezifischen Infusionsparameters zugeordnet ist.

7. Vorrichtung nach irgendeinem der Ansprüche 1 bis 6, dadurch gekennzeichnet, daß die Anzeigeeinheit (54) Mittel umfaßt, um spezifische Infusionsparameter anzuzeigen, die von den in die Speichereinheit der netzunabhängigen Überwachungseinheit geladenen spezifischen Infusionsparametern abgeleitet werden.

8. Vorrichtung nach Anspruch 7, dadurch gekennzeichnet, daß die angezeigten spezifische Parameter Infusionsvolumen, Infusionsdauer und Toleranzbereiche umfaßen.

9. Vorrichtung nach irgendeinem der Ansprüche 1 bis 8, dadurch gekennzeichnet, daß die Anzeigeeinheit (54) Mittel umfaßt, um auf einer linearen Skala die positive oder negative Abweichung der Anzahl während eines beweglichen Beobachtungszeitraums t1 erfassten Tropfen von der Anzahl der Tropfen, die während dieses Zeitraums t1 bei einer einheitlichen Verteilung der Tropfen auf eine zu Beginn vorgegebene Infusionsdauer vorliegen müsste, graphisch darzustellen.

10. Vorrichtung nach Anspruch 9, dadurch gekennzeichnet, daß die erste Recheneinheit dazu geeignet ist, eine optimale Tropfenanzahl ständig zu berechnen, die auf der linearen Skala in Form eines Cursors (128) angezeigt wird, der die relative Abweichung dieser optimalen Tropfzahl gegenüber der Anzahl, die der Nullabweichung entspricht, angibt, und die folgendermaßen definiert wird:

$$\frac{(\Sigma \text{ der Tropfen, die durchlaufen sollen}) - (\Sigma \text{ bereits durchgelaufener Tropfen})}{(\text{Gesamtdauer der Infusion}) - (\text{bisherige Infusionsdauer})}$$

multipliziert mit der Dauer t1 des beweglichen Beobachtungszeitraums.

11. Vorrichtung nach irgendeinem der Ansprüche 1 bis 10, dadurch gekennzeichnet, daß der Sensor (14) eine Infrarotstrahlungsquelle (140), die impulsförmig betrieben wird, sowie einen optischen Empfänger, der damit synchron aktiviert wird, umfaßt.

12. Vorrichtung nach Anspruch 11, dadurch gekennzeichnet, daß dieser optische Empfänger mehrere Photodioden (142) umfaßt, die elektrisch in Serie geschaltet sind und in einem Winkelsektor gegenüber der Infrarotstrahlenquelle verteilt sind.

13. Vorrichtung nach Anspruch 11 oder 12, dadurch gekennzeichnet, daß der Sensor (14)

einen integrierenden Verstärker (148), um das Signal des optischen Empfängers zu integrieren und als Ausgangssignal einen um einen regelbaren Faktor verstärkten Mittelwert zu liefern; sowie einen Vergleicher (150), um dieses Ausgangssignal mit dem nicht integrierten Signal des Empfängers zu vergleichen und ein logisches Signal (83) als Eingangssignal für die Einheit zur zeitlichen Erfassung und Zählung (84) zu liefern; umfaßt.

14. Vorrichtung nach irgendeinem der Ansprüche 1 bis 13, dadurch gekennzeichnet, daß zur vorübergehenden Ankopplung der Eingangsschnittstelle der Überwachungseinheit an die Ausgangsschnittstelle der Programmierkonsole ein optoelektronisches Koppelelement (15) verwendet wird.

15. Vorrichtung nach irgendeinem der Ansprüche 1 bis 14, dadurch gekennzeichnet, daß die netzunabhängige Überwachungseinheit (12) außerdem

Mittel zur Umschaltung (94), um von einem Betriebsmodus Überwachung/Alarm auf einen Betriebsmodus Mengeneinstellung umzuschalten, in dem akustische Alarme außer Betrieb sind; sowie eine Akustikeinheit, die dazu geeignet ist, in diesem Betriebsmodus "Mengeneinstellung" ein akustisches Signal für jeden erfassten Tropfen zu erzeugen; umfaßt.

16. Vorrichtung nach irgendeinem der Ansprüche 1 bis 15, dadurch gekennzeichnet, daß die netzunabhängige Überwachungseinheit (12) außerdem Mittel umfaßt, um die erste Recheneinheit (88) durch Anhalten ihres Oszillators (96) zwischen aufeinanderfolgenden Ereignissen außer Betrieb zu setzen, wobei ein Ereignis mit dem Durchlauf eines Tropfens, dem Überschreiten der Zeitgrenze zwischen zwei Tropfen oder dem Ausfall des Sensors gleichgesetzt wird.

17. Vorrichtung nach Anspruch 16), dadurch gekennzeichnet, daß die Einheit zur zeitlichen Erfassung und Zählung (84)

erste logische Mittel (108), um einen Übergang eines ersten logischen Signals bei einem Ereignis ersten Typs zu erzeugen, das entweder dem Durchlauf eines Tropfens oder dem Überschreiten der Zeitgrenze zwischen zwei Tropfen oder auch einem Ausfall des Sensors entspricht, wobei diese ersten logischen Mittel den Oszillator (96) der Recheneinheit (88) bei einem Ereignis ersten Typs wieder in Gang setzen;

zweite logische Mittel (106), um einen Übergang eines zweiten logischen Signals ausschließlich bei Durchlauf eines Tropfens zu erzeugen, wobei diese zweiten logischen Mittel (106) in der Recheneinheit (88) bei Durchlauf eines Tropfens den Ablauf eines Rechenalgorithmus auslösen; sowie

Mittel zur Erzeugung eines Alarms bei Übergang des ersten logischen Signals und Nicht-Übergang des zweiten logischen Signals;

umfaßt.

18. Vorrichtung nach Anspruch 16 oder 17, dadurch gekennzeichnet, daß die Einheit zur zeitlichen Erfassung und Zählung (84) außerdem

einen Impulszähler (102) zum Zählen der Impulse einer Niederfrequenzuhr (100);

einen Verschlußzähler (104), der diesem Impulszähler (102) beigeordnet ist und der so an die Recheneinheit (88) angeschlossen ist, daß er von ihr abgefragt werden kann;

umfaßt;

wobei die ersten logischen Mittel so an den Impulszähler (102) und an den Verschlußzähler (104) angeschlossen sind, daß sie bei Übergang des ersten logischen Signals das Abzählen der Impulse in dem Verschlußzähler (104) stoppen und den Impulszähler (102) auf Null stellen.

19. Vorrichtung nach Anspruch 18, dadurch gekennzeichnet, daß die Einheit zur zeitlichen Erfassung und Zählung (84) außerdem

einen Vergleicher (110), der den Wert des Impulszählers (102) ständig mit einem vorher festgelegten Grenzzeitraum vergleicht, umfaßt

wobei dieser Vergleicher (110) bei Überschreiten dieses vorher festgelegten Grenzzeitraums einen Übergang des ersten logischen Signals auslöst.

Fig.1

EP 0 797 462 B1

**Fig.2**

**Fig.3**

Fig.4

Fig. 5

EP 0 797 462 B1

Fig. 6

Fig. 7

Fig. 8

122

120 →

| VOLUME = 525 ml | TEMPS = 04 H 30 mn | - 20 %   + 15% |

% injecté

132

0    50    100

REGLAGE

128

DEBIT
INSTANTANE

124

ecart %    -20   -10   0   +10   +20

126

DEBIT
MOYEN

130

*Fig.9*